# EUROPÄISCHE PATENTSCHRIFT

(11) **EP 1 637 080 B1**
(45) Veröffentlichungstag und Bekanntmachung des Hinweises auf die Patenterteilung: **02.03.2011**
(21) Anmeldenummer: 04022385.1
(22) Anmeldetag: 21.09.2004
(51) Int. Cl.: A61B 6/10

(54) **Vorrichtung zum Vermeiden von Kollisionen mit einem bildgebenden medizinischen Gerät**
System for preventing collisions with a medical imaging device
Système d'anti collision avec un appareil d'imagerie médicale

(43) Veröffentlichungstag der Anmeldung: 22.03.2006
(73) Patentinhaber: BrainLAB AG, 85622 Feldkirchen (DE)
(72) Erfinder: Bauch, Thomas, 80469 München (DE); Weissenborn, Anke, 80805 München (DE)
(74) Vertreter: Schwabe - Sandmair - Marx

(56) Entgegenhaltungen:
- EP-A- 0 459 717
- US-A- 2 844 349
- US-A- 4 578 757
- US-A- 5 651 044
- US-A- 5 654 997
- US-A- 5 677 535
- US-A- 5 883 935
- US-A1- 2003 069 653

## Beschreibung

Die Erfindung betrifft eine Sicherheitsvorrichtung für ein bildgebendes medizinisches Gerät, insbesondere für einen Computertomographen, einen Kernspintomographen oder ein PET-Gerät. Insbesondere betrifft sie eine Bremsvorrichtung für ein System aus einem solchen bildgebenden medizinischen Gerät und einem Patiententräger und derartige Geräte und Systeme mit Sicherheitsvorrichtungen bzw. Bremsvorrichtungen.

Bildgebende medizinische Geräte, wie sie gemäß der vorliegenden Erfindung in Betracht zu ziehen sind, werden manchmal als mobile Geräte bereitgestellt, die über einen Patiententräger gefahren werden können, auf dem ein Patient liegt. Andererseits gibt es auch bildgebende medizinische Geräte, in die ein Patiententräger mit darauf liegendem Patienten einfährt. In beiden Fällen findet eine Relativbewegung zwischen dem Patiententräger (dem Patienten) und dem Gehäuse des Geräts statt, welches im weiteren auch als "Gantry" bezeichnet wird.

Solche Gantrys weisen einen Eintritt für den Patiententräger und den auf ihm liegenden Patienten auf. Dieser Patienteneintritt hat meist die Form einer runden Öffnung an einer Stirnseite der Gantry.

Aus den Druckschriften US-A-5,654,997, US-A-5,651,044, EP-A-0 459 717, US-A-5,677,535, US-A-5,883,935 und US-2003/069653 A1 sind bildgebende Geräte in Form von C-Bögen bekannt, welche Näherungs- oder Berührungsdetektoren im Bereich des Strahlungsempfängers aufweisen. Anhand dieser Sensoren soll eine Kollision des Strahlungsempfängers mit dem Patienten verhindert werden.

Aus der EP 1 262 146 A2 ist ein CT-Gerät oder ein Kernspintomographiegerät mit einer runden Öffnung als Patienteneintritt bekannt.

Obwohl es für mobile, also selbstverfahrbare Gantrys im Fußbereich schon Kontaktbremsen gibt, welche die Gantry in ihrer Gleitbewegung bzw. Fahrbewegung abbremsen bzw. stoppen, wenn die Gantry selbst im Fußbereich an einen Widerstand stößt, sind Sicherungsvorrichtungen für den Patienteneintritt der Gantry bisher nicht in Betracht gezogen worden.

Es ist die Aufgabe der vorliegenden Erfmdung, eine Sicherheitsvorrichtung für ein bildgebendes medizinisches Gerät bereit zu stellen, welche es möglich macht, das Gerät im Bereich des Patienteneintritts zu sichern.

Diese Aufgabe wird erfindungsgemäß durch eine Sicherheitsvorrichtung gemäß dem Anspruch 1 gelöst. Gemäß der Erfindung wird im Bereich des Patienteneintritts in Form einer Munden Öffnung ein Näherungs- oder Berührungsdetektor angeordnet. Durch den Detektor kann ermittelt werden, ob im Bereich des Patienteneintritts, also dort wo empfindlichere Teile des medizinischen Gerätes liegen, eine unerwünschte Annäherung oder Berührung von außen stattfindet. Wenn eine solche Annäherung oder Berührung stattfindet, können dann entsprechende Maßnahmen getroffen werden, um Beschädigungen oder eine falsche Handhabung zu vermeiden.

Ferner liegt der Detektionsbereich des Näherungs- oder Berührungsdetektors in der Ebene des Patienteneintritts und/oder steht mindestens teilweise aus der Ebene vor. Damit können Näherungen bzw. Berührungen detektiert werden, bevor tatsächlich ein Kontakt mit der Gantry im Eintrittsbereich stattfindet.

Der Detektor ist ganz (bevorzugt ringförmig) oder abschnittsweise um den Patienteneintritt herum angeordnet. Mit einer solchen Anordnung wird eine entsprechende Sicherheit an jeder Stelle des Patienteneintritts gewährleistet.

Die Erfindung betrifft ferner eine Bremsvorrichtung für ein System aus einem bildgebenden medizinischen Gerät, insbesondere einem Computertomographen oder einem Kernspintomographen, und einem Patiententräger, die relativ zueinander so bewegbar sind, dass der Patient in den Patienteneintritt des Geräts eingefahren werden kann. Die Bremsvorrichtung umfasst eine Sicherheitsvorrichtung, wie sie oben in verschiedenen Ausführungsformen beschrieben wurde, wobei der Näherungs- oder Berührungsdetektor bei einer unerwünschten, z.B. zu weit gehenden Annäherung oder Berührung durch den Patienten oder den Patiententräger ein Bremssignal erzeugt, welches mittels einer Bremse die Relativbewegung zwischen dem Gerät und dem Patiententräger bremst. Auf diese Weise können Kollisionsschäden am Gerät und am Patiententräger und vor allem auch Verletzungen des Patienten vermieden werden. Wenn das System ein bewegliches medizinisches Gerät aufweist, kann das Gerät abgebremst werden. Wenn das System einen beweglichen Patiententräger aufweist, kann der Patiententräger abgebremst werden.

Ferner betrifft die Erfindung noch ein bildgebendes medizinisches Gerät, insbesondere einen Computertomographen, einen Kernspintomographen oder ein PET-Gerät, mit einer Sicherheitsvorrichtung wie sie oben beschrieben worden ist.

Außerdem betrifft die Erfindung ein System aus einem bildgebenden medizinischen Gerät, insbesondere einem Computertomographen, einem Kernspintomographen oder einem PET-Gerät, und einem Patiententräger, wobei das System eine Bremsvorrichtung aufweist, wie sie oben beschrieben worden ist.

Die Erfindung wird im weiteren anhand der beiliegenden Zeichnungen näher erläutert. Sie kann alle hierin beschriebenen Merkmale wie in den Ansprüchen definiert aufweisen. In den Zeichnungen zeigen:
- Figur 1: eine schematische Stirnansicht einer CT-Gantry mit einer erfindungsgemäßen Sicherheitsvorrichtung; und
- Figur 2: eine schematische Seitenansicht eines Gantry-Patiententräger-Systems mit einer Sicherheitsvorrichtung gemäß der vorliegenden Erfindung.

Die in den Figuren dargestellte Computertomographen-Gantry hat das Bezugszeichen 1, und sie ist nur schematisch gezeigt. Sie weist den Patienteneintritt 2 auf, im vorliegenden Fall eine kreisrunde Öffnung, hinter der sich die Bildaufnahmeelemente des Computertomographen befinden.

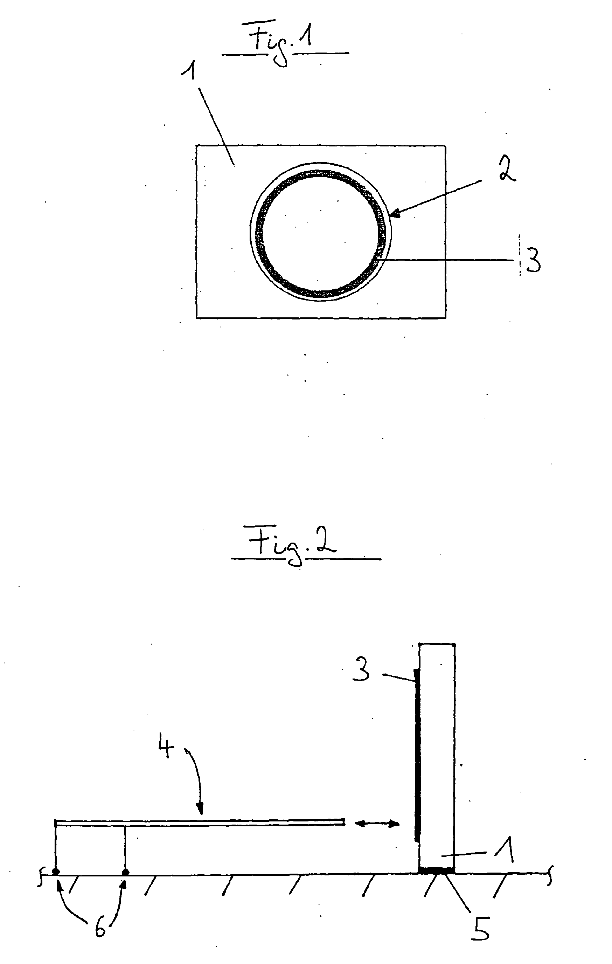

Um die kreisrunde Öffnung des Patienteneintritts 2 herum, etwas nach innen verlagert und, wie aus Figur 2 hervorgeht, etwas nach vorne von der Gantry 1 abstehend, ist der Berührungsdetektor-Ring 3 angebracht. Er ist in bekannter Weise ausgestaltet und muss daher nicht näher beschrieben werden. Seine Funktion ist die, dass er in dem Fall, wo ein festes Objekt einen Kontakt mit dem Berührungsdetektor 3 macht, ein Signal abgeben kann, welches dann auf verschiedenste Weise in sicherheitsrelevanter Art genutzt werden kann.

Das oben genannte Signal kann beispielsweise einen Bremsvorgang auslösen, d.h. eine Relativbewegung zwischen der Gantry 1 und einem Patiententräger 4 bremsen bzw. anhalten, wenn der Patiententräger 4 oder der auf ihm liegende Patient an den Berührungsdetektor 3 stößt.

In Figur 2 sind alle möglichen Fälle der Relativbewegung gezeigt. Die Gantry 1 kann beispielsweise einen Fahr-/Gleitmechanismus 5 aufweisen, oder der Patiententräger 4 kann einen Fahr-/Gleitmechanismus 6 aufweisen. Beide Mechanismen 5 und 6 können von jedweder Art sein und sind in Figur 2 nur schematisch dargestellt.

Der Berührungsdetektor 3 kann das Signal intern (mit einer Signalleitung oder drahtlos) an den Fahrmechanismus 5 bzw. an die Bremse für den Fahrmechanismus 5 weitergeben, um eine Bremsung einzuleiten. Dies gilt für den Fall, dass die Gantry 1 selbst verfahren wird. Das Signal des Berührungsdetektors 3 kann aber auch per Leitung oder drahtlos an den Fahrmechanismus 6 für den Patiententräger 4 übertragen werden, um die Bremsung dort einzuleiten, wenn der Patiententräger 4 selbst verfahrbar ist.

Anstelle des Berührungsdetektors kann auch ein Näherungsdetektor verwendet werden, der zum Beispiel eine Art Lichtschranke umfassen kann. Jede Art bekannter Näherungsdetektoren ist einsetzbar.

## Patentansprüche

1. Sicherheitsvorrichtung für ein bildgebendes medizinisches Gerät (1), das eine runde Öffnung als Patienteneintritt (2) aufweist, wobei ein einen Detektionsbereich aufweisenden Näherungs- oder Berührungsdetektor (3) ganz oder abschnittsweise um den Patienteneintritt (2) herum angeordnet ist, und wobei der Detektionsbereich des Näherungs- oder Berührungsdetektors (3) in einer Ebene des Patienteneintritts (2) liegt und/oder mindestens teilweise aus der Ebene vorsteht.

2. Sicherheitsvorrichtung nach Anspruch 1, **dadurch gekennzeichnet, dass** der Detektor (3) ringförmig um den Patienteneintritt (2) herum angeordnet ist.

3. Bremsvorrichtung für ein System aus einem bildgebenden medizinischen Gerät (1), das eine runde Öffnung als Patienteneintritt aufweist, insbesondere einem Computertomographen, einem Kernspintomographen oder einem PET-Gerät, und einem Patiententräger (4), die relativ zueinander so bewegbar sind, dass der Patient in den Patienteneintritt (2) des Geräts (1) eingefahren werden kann, **dadurch gekennzeichnet, dass** die Bremsvorrichtung eine Sicherheitsvorrichtung nach einem der Ansprüche 1 oder 2 aufweist, wobei der Näherungs- oder Berührungsdetektor (3) bei einer Annäherung oder Berührung durch den Patienten oder den Patiententräger ein Bremssignal erzeugt, welches mittels einer Bremse die Relativbewegung zwischen dem Gerät (1) und dem Patiententräger (4) bremst.

4. Bremsvorrichtung nach Anspruch 3, **dadurch gekennzeichnet, dass** bei einem System mit einem beweglichen medizinischen Gerät (1) das Gerät (4) abgebremst wird.

5. Bremsvorrichtung nach Anspruch 3, **dadurch gekennzeichnet, dass** bei einem System mit einem beweglichen Patiententräger (4) der Patiententräger (4) abgebremst wird.

6. Bildgebendes medizinisches Gerät, das eine runde Öffnung als Patienteneintritt aufweist, insbesondere ein Computertomograph, ein Kernspintomograph oder ein PET-Gerät, mit einer Sicherheitsvorrichtung bzw. Bremsvorrichtung nach einem der Ansprüche 1 bis 3.

7. System aus einem bildgebenden medizinischen Gerät, das eine runde Öffnung als Patienteneintritt aufweist, insbesondere einem Computertomographen, einem Kernspintomographen oder einem PET-Gerät, und einem Patiententräger, **dadurch gekennzeichnet, dass** es eine Bremsvorrichtung nach einem der Ansprüche 3 bis 5 aufweist.

## Claims

1. A safety device for an imaging medical apparatus (1) comprising a round opening as a patient entrance (2), wherein a proximity or contact detector (3) comprising a detection range is arranged around the patient entrance (2), completely or in sections, and wherein the detection range of the proximity or contact detector (3) is in a plane of the patient entrance (2) and/or at least partially protrudes out of said plane.

2. The safety device according to claim 1, **characterised in that** the detector (3) is arranged around the patient entrance (2) annularly.

3. A braking device for a system consisting of an imaging medical apparatus (1) comprising a round opening as a patient entrance, in particular a computer tomograph, a nuclear spin tomograph or a PET apparatus, and a patient carrier (4) which can be moved relative to each other such that the patient can be moved into the patient entrance (2) of the apparatus (1), **characterised in that** the braking device comprises a safety device according to any one of claims 1 or 2, wherein in the event of proximity or contact by the patient or patient carrier, the proximity or contact detector (3) generates a braking signal which brakes the relative movement between the apparatus (1) and the patient carrier (4) by means of a brake.

4. The braking device according to claim 3, **characterised in that** in a system comprising a mobile medical apparatus (1), the apparatus (4) is braked.

5. The braking device according to claim 3, **characterised in that** in a system comprising a mobile patient carrier (4), the patient carrier (4) is braked.

6. An imaging medical apparatus comprising a round opening as a patient entrance, in particular a computer tomograph, a nuclear spin tomograph or a PET apparatus, comprising a safety device and/or braking device according to any one of claims 1 to 3, respectively.

7. A system consisting of an imaging medical apparatus comprising a round opening as a patient entrance, in particular computer tomograph, a nuclear spin tomograph or a PET apparatus, and a patient carrier, **characterised in that** it comprises a braking device according to any one of claims 3 to 5.

## Revendications

1. Dispositif de sécurité pour un appareil d'imagerie médicale (1), comportant une ouverture ronde servant d'entrée (2) pour un patient, dans lequel un détecteur de proximité ou de contact (3) comportant une zone de détection est agencé entièrement ou par sections autour de l'entrée du patient (2), et dans lequel la zone de détection du détecteur de proximité ou de contact (3) se situe dans un plan de l'entrée du patient (2) et/ou fait saillie au moins partiellement à partir du plan.

2. Dispositif de sécurité selon la revendication 1, **caractérisé en ce que** le détecteur (3) est agencé autour de l'entrée de patient (2) de manière annulaire.

3. Dispositif de freinage pour un système constitué d'un appareil d'imagerie médicale (1) comportant une ouverture ronde servant d'entrée pour un patient, en particulier un tomographe informatique, un tomographe à résonance magnétique nucléaire ou un appareil PET, et d'un support de patient (4), qui sont mobiles l'un par rapport à l'autre de telle sorte que le patient peut être introduit dans l'entrée de patient (2) de l'appareil (1), **caractérisé en ce que** le dispositif de freinage comporte un dispositif de sécurité selon l'une quelconque des revendications 1 ou 2, dans lequel le détecteur de proximité ou de contact (3) génère un signal de freinage en cas d'approche du patient ou du support de patient ou de contact avec celui-ci, lequel signal ralentit le déplacement relatif entre l'appareil (1) et le support de patient (4) au moyen d'un frein.

4. Dispositif de freinage selon la revendication 3, **caractérisé en ce que** l'appareil (4) est freiné avec un système muni d'un appareil médical mobile (1).

5. Dispositif de freinage selon la revendication 3, **caractérisé en ce que** le support de patient (4) est freiné avec un système muni d'un support de patient mobile (4).

6. Appareil d'imagerie médicale comportant une ouverture ronde servant d'entrée pour un patient, en particulier un tomographe informatique, un tomographe à résonance magnétique nucléaire ou un appareil PET, muni d'un dispositif de sécurité ou d'un dispositif de freinage selon l'une quelconque des revendications 1 à 3.

7. Système constitué d'un appareil d'imagerie médicale comportant une ouverture ronde servant d'entrée pour un patient, en particulier un tomographe informatique, un tomographe à résonance magnétique nucléaire ou un appareil PET, et d'un support de patient, **caractérisé en ce qu'**il comporte un dispositif de freinage selon l'une quelconque des revendications 3 à 5.
